# EUROPEAN PATENT SPECIFICATION

(11) **EP 1 446 172 B1**
(45) Date of publication and mention of the grant of the patent: **14.04.2010**
(21) Application number: 02761817.2
(22) Date of filing: 06.09.2002
(51) Int. Cl.: A61M 15/00

(54) **Inhalation device type DPI**
Inhalator Typ DPI
Inhalateur type DPI

(30) Priority: 06.09.2001 US 317706 P
(43) Date of publication of application: 18.08.2004
(73) Proprietor: MicroDose Therapeutx, Inc., Monmouth Junction, NJ 08852 (US)
(72) Inventor: Abrams, Andrew, L., Westport, CT 06880 (US); Gumaste, Anand, V., Princeton Junction, NJ 08550 (US)
(74) Representative: Prinz & Partner
(86) International application number: PCT/US2002/030308
(87) International publication number: WO 2003/022332

(56) References cited:
- EP-A- 0 667 168
- EP-A- 0 933 092
- WO-A-01/19436
- WO-A-97/18003
- US-A- 5 794 612
- US-A- 5 839 430
- US-A- 6 158 676
- US-B1- 6 240 917

## Description

The present invention relates generally to the field of dry powder inhalation devices, and more specifically, to a mouthpiece adaptor and patient feedback for an inhaler. The invention has particular utility as a mouthpiece adaptor and patient feedback for dry powder inhalers ("DPIs") for facilitating use of same by pediatric, geriatric and compromised patients.

Metered dose inhalers (MDIs) depend on delivery technique to deliver the proper amount of medication to the lungs. A properly delivered MDI medication depends on dexterity, coordination, timing, and practice. This can be a real problem when the patient is young, has coordination problems, or especially irritable airways.

An alternative to MDIs are dry powder inhaler devices (DPIs), which are activated by the patient's inspiratory effort, so that coordination is not a problem, although inhalation technique is still important. The drug is aerosolized by the airflow through a DPI created by the patient inhaling. DPI devices are easy to use and are thus suitable for most ages. Newer multidose types of powder devices have electronics which include dose counters, which enable patients to check whether or not they have taken a dose and warns them when the inhaler is running out of doses by showing them exactly how many doses remain. U.S. Patent 6,142,146 discloses this type of device. However, dry powder inhalers require patients to inspire reasonably rapidly to inhale the dry powder, so these devices are not suitable for younger children and infants. Further, even if young children and infants could conform to the protocol for using the device, current devices have mouthpieces which are too cumbersome for these patients.

When dealing with bronchial diseases among children and infants, it is difficult to make the patient properly inhale the therapeutic substances necessary for treatment When asthma makes its debut among infants and young children, typically at 8 months to 2.5 years, it is especially difficult to make a child or infant inhale the prescribed medical substances in the proper way. Children and infants have limited lung capacity and the force of a child's or infant's breath during inhalation (inhalation flow) is thus limited. This is even more apparent when the child or infant is suffering from asthma or other bronchial diseases. Parents also desire that the devices used for inhalation be as flexible as possible, as it is difficult to position the inhaler in a way that will allow proper inhalation by an infant.

For patients of limited or compromised inhalation capacity, inhalation therapy may be accomplished through the use of inhalation chambers. An inhalation chamber typically includes an inlet and fixture for a medicament dispenser, e.g., an MDI, and an expanded hollow body, which in the technical field of inhalers normally is called a "spacer" or inhalation chamber, having an outlet provided at the end remote from the inlet. An inhalation/exhalation valve, e.g., a one-way valve, typically is provided adjacent the outlet, and a mouthpiece is provided at the outlet. When such a device is used by older children or adults the mouthpiece is inserted between the teeth and the lips are closed around the mouthpiece. It is, however, not possible for young children and infants to hold such a mouthpiece between their lips. Moreover, these devices are constructed to be used by older children who have large lung capacity and who can inhale more forcefully. The inhalation/exhalation valves provided typically require a certain inhalation flow, which a child or infant is unable to generate, to open properly. Therefore, for satisfactory inhalation to be achieved by young children and infants, inhalation devices often are provided with a face mask.

However, some small children and infants find standard inhalation devices employing masks frightening, and, as a consequence; resist using them. Faced with strong resistance from children, many care-givers responsible for administering medication to children report a reluctance to offer air-borne drugs for use with standard inhalation devices employing masks on a regular basis. In addition, care-givers also report that even when attempted, the delivery of aerosol/gas medication to children is often sub-optimal because the child cries and/or forcibly removes the mask from their face before the medication is taken properly.

Many patients who cannot use MDIs or DPIs, even with inhalation chambers, are forced to use nebulizers. Nebulizers produce a cloud of medication by passing a jet of compressed air through a solution of a drug (jet nebulizers) or by dropping the drug solution onto a plate vibrating at high frequency (ultrasonic nebulizers). Nebulizers have the advantage of being able to be used by patients of all ages, including young babies, because coordination is unimportant. However, disadvantages of nebulizers include that they are cumbersome, expensive (both the machine and the drugs) and noisy; require a power source, typically lines (AC) current; treatment takes a long time, often around ten minutes; and young patients are required to wear a mask. The amount of drug delivered to the lungs is highly dependant on the breathing pattern of the patient. All these factors tie down the patient and care-giver.

Because nebulizers have the above problems, several devices have been developed in order to entice or teach children how to use MDIs or DPIs, so that a nebulizer is unnecessary. But, those devices designed for use by young children require a mask adaptor for the MDI or DPI. PCT Application No. 995398 and European Patent 667168 disclose incentive systems of this type.

Quite apart from the foregoing, conventional MDIs and DPIs (and also inhalation chambers therefor) have mouthpieces sized for the adult population. Thus, conventional MDIs, DPIs and inhalation chambers therefor have mouthpieces that are too big to be comfortably used by small children and infants. Moreover, the mouthpieces of conventional MDIs, DPIs and spacers are primarily rectangular in shape, which is somewhat awkward, or at least unfamiliar, particularly to small children.

WO-A-97/18003 A1 discloses an air flow system for detecting target flow rates as a function of an opening in a flow restrictor. The airflow system includes a patient mouthpiece and at least an interchangeable flow restrictor. The flow resistor has a tapered opening sized for frictional engagement with one end of a T-tube. At base of the opening is a pressure sensing port. A tube is pressure-fit into the port and protrudes slightly above the inside surface of the opening. The protrusion of the tube serves as a stop for the connection with the T-tube. The opposite end of the flow restrictor has an external taper for frictional engagement with another flow restrictor.

WO-A-01/19436 A1 shows a combination mouthpiece for inhalation therapy devices, having a body defining an aerosol duct through which, in use, a stream of air carrying an aerosolized drug is supplied and an inlet hole through the body for receiving an oxygen tube adapter. At least one partition of the body defines a oxygen duct, the oxygen duct extending generally parallel with the aerosol duct.

US 6,158,676 teaches micro-atomizing device which utilizes vortex energy in a maximized, high energy density, stable force field.

EP 0 667 168 A1 relates to a metered dose inhaler including a source of airborne medication for inhalation by a patient, a flow measurement means coupled with the inhaler means for measuring the flow of air through the inhaler means, a microprocessor means for integrating the flow of air over a time to produce a measure of volume inhaled, storage means for storing the measured flow and volume values and for storing additional quantifiable diagnostic parameters related to projected patterns of medication distribution to receptor sides in a patient's lungs, and display means for providing a visual display of measured flow and volume values of the patient as well as said diagnostic parameters related to projected delivery of medication.

Finally, EP 0 933 092 A1 relates to an aerosol dispensing inhaler training device for determining whether a user is properly operating an aerosol dispensing device.

This invention seeks to solve the problems with the prior art by allowing young children, geriatric and compromised patients to use DPIs mask-free. Another aspect of the invention seeks to solve the issue of consistent patient inhalation in the pediatric, geriatric or physically challenged patients. Another aspect of the invention seeks to solve the problem of patient compliance when the patient is young, thereby enabling them to use certain devices previously unavailable to them. Yet another aspect of the invention seeks to allow sick or infirm patients to receive a dosage or medicine through an extension to the mouthpiece, thereby allowing the patient to recline while receiving medicine through an inhaler.

These objects are solved, at least in part, by a dry powder inhaler according to claim 1.

Still other features and advantages of the present invention may be seen from the following detailed description taken in connection with the attached drawings wherein like numerals depict like parts, and wherein:
Figure 1 is a longitudinal view of an exemplary adaptor for an inhaler attached to an inhaler according to one embodiment of the invention;
Figure 2 is a longitudinal view of an exemplary adaptor for an inhaler attached to an inhaler according to another embodiment of the invention;
Figure 3 is a longitudinal view of an exemplary adaptor for an inhaler attached to an inhaler according to yet another embodiment of the invention;
Figure 4 is a frontal view of an exemplary adaptor for an inhaler and a mouthpiece for an adaptor according to one embodiment of the invention;
Figure 5 is a frontal view of an exemplary adaptor for an inhaler and the mouthpiece of the adaptor according to another embodiment of the invention;
Figure 6 is a longitudinal view of an exemplary adaptor for an inhaler attached to the inhaler according to yet another embodiment of the invention;
Figure 7 is a functional block diagram of the sensor-light/sound mechanism of the invention according to one embodiment of the invention;
Figure 8 is a longitudinal view of an exemplary adaptor employing the sensor-light/sound mechanism of Figure 7; and
Figure 9 is a frontal view of an exemplary adaptor with a sensor-light/sound mechanism according to the embodiment shown in Figure 8.

The detailed description of the preferred embodiments illustrates the use of the adaptor with DPIs.

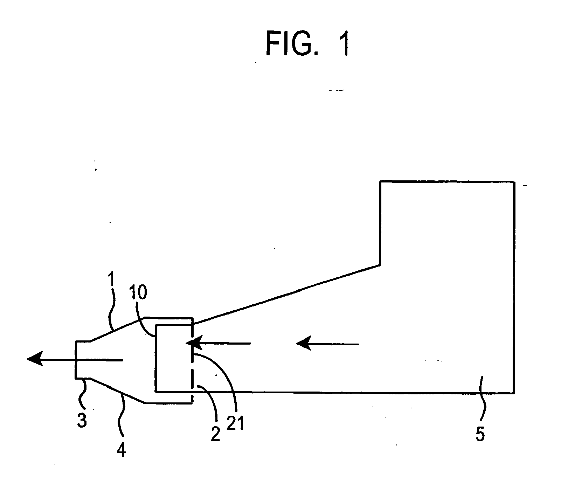

Figure 1 illustrates one embodiment of an exemplary adaptor, wherein an adaptor 1 is attached to the mouthpiece 10 of an inhaler 5. The adaptor is attached to the inhaler mouthpiece 10 from an attachment side 2 using an attachment means that forms an

Therefore, the invention provides a mouthpiece adaptor for a dry powder or metered dose inhaler comprising a connecting side for direct connection to a mouthpiece of an inhaler; a mouthpiece side of reduced size relative to the inhaler mouthpiece; and a transition section between said connecting side and mouthpiece side, **characterized in that** the mouthpiece side has a diameter of from 1 to 15 mm, and the inner lumen of the adaptor is smoothly tapered from the connecting side to the mouthpiece side whereupon airflow may be concentrated with minimum turbulence.

Preferred embodiments of the invention are characterized by the additional features of appending claims 2-5.

airtight seal. In this embodiment, the adaptor side is designed to create a friction fit with the inhaler mouthpiece. The friction fit is accomplished by creating the attachment side of the adaptor to be just slightly larger in inside dimension than the outside dimension of the inhaler mouthpiece to which it is designed to attach, and sliding the adaptor onto the inhaler mouthpiece, thereby creating an airtight seal.

The adaptor I also includes a transition section 4 between a mouthpiece side 3 and an attachment side 2. The transition section 4 tapers the attachment side 2 of the adaptor to a child-sized mouthpiece side. Aerated particles enter and immediately pass through the connection means from the inhaler mouthpiece upon proper stimulation of the inhalation device. The connecting means does not act as a holding chamber. Here, the connection means is tapered in a funnel-like shape to minimize turbulence and prevent the coagulation of the aerated particles upon usage of the adaptor, which helps maintain the inhaler's effectiveness.

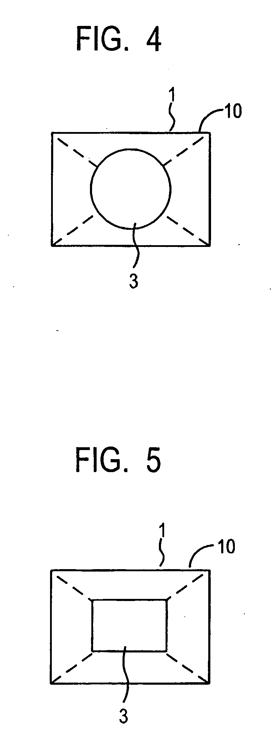

The mouthpiece side 3 is child-sized, similar in cross-sectional dimension to that of a drinking straw, i.e., having a diameter in the range of 1mm to 15mm, and is either round or oval shaped, as shown in Figure 4. A round shape for the mouthpiece side is preferred for young children who are already familiar with this shape, i.e., from the use of drinking straws. That is to say, young children are already familiar with using a straw, and have shown that they are capable of proper inspirator pressures. Thus, the mouthpiece side straw-like size and shape not only encourages patient compliance, but also presents a familiar, less frightening alternative to a mask.

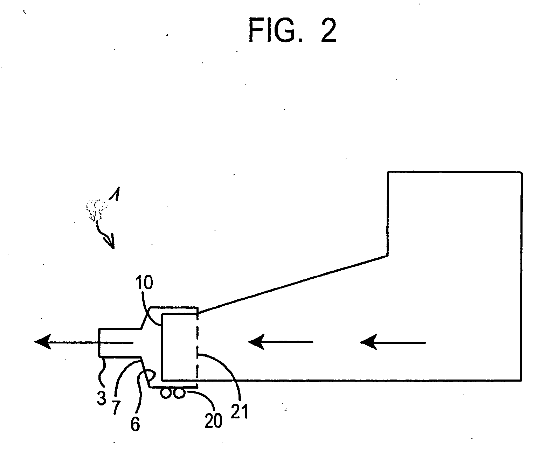

A second embodiment of mouthpiece adaptor 1 is illustrated in Figure 2. This embodiment, like the first, includes a connecting side 2, a transition section 4, and a mouthpiece side 3. As in the first embodiment, the connecting side 21 must facilitate the formation of an airtight seal. As shown in Figure 2, this is accomplished by securing a plastic or metal clamp 20, which is located on the connecting side 21 of the mouthpiece adaptor. Here, the mouthpiece adaptor attachment side 2 is larger in inside dimension than the outside dimension of the inhaler mouthpiece 10, and allows the adaptor to easily slide on the inhaler mouthpiece 10. Once the mouthpiece adaptor 1 is slid over the mouthpiece of the inhaler 10, the clamps 20 are fastened, creating the requisite airtight seal.

The mouthpiece adaptor of Figure 2 includes a funnel-like region between the connecting side 21 and the mouthpiece side 3 comprising a slanted wall 7 formed opposite of the inhaler mouthpiece. A metal foil 6 is applied to the slanted wall connected to the circuit of the MDI or DPI and charged by the power source of the MDI or DPI, to the same charge as the particles. This causes a repulsion between the slanted wall and the particles whereby to prevent the particles from collecting on or colliding with the wall, and thus prevent coagulation of the particles on the interior adaptor walls. Further, as with the adaptor of Figure 1, aerated particles only enter and pass through the adaptor of Figure 2 upon proper stimulation of the inhalation device, i.e., the connection means does not act as a holding, chamber.

The mouthpiece side of the adaptor of Figure 2, as illustrated in Figure 5, is manufactured as a replication of the inhaler mouthpiece. Like the mouthpiece side in the adaptor of Figure 1, the mouthpiece side in the adaptor of Figure 2 is child-sized, i.e., having cross-sectional dimensional area from 1mm² to 200mm². This design, like the round or cylindrical mouthpiece side, also has advantages. Because the shape of the mouthpiece side here is the shape of the inhaler mouthpiece, airflow is concentrated with minimum turbulence.

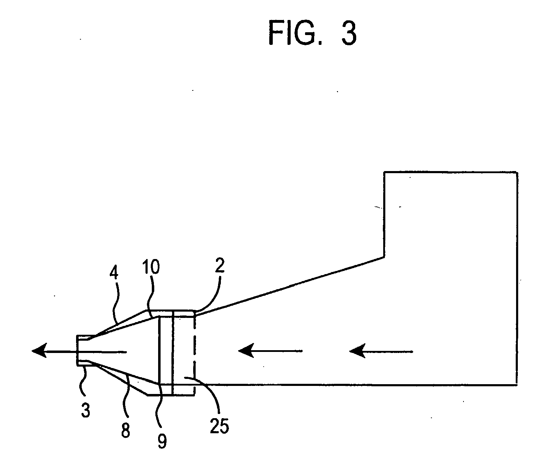

A third embodiment is shown in Figure 3. The third embodiment, like the first and second, includes an attachment side 2, a transition section 4, and a mouthpiece side 3. Here, the attachment side 2 is fitted to the inhaler mouthpiece 10 using an attachment means that employs a rubber ring to create an airtight seal. The connecting side 2 is slightly larger than the inhaler mouthpiece 10, and includes on the distal end a taut rubber ring 25 fitted over the connecting side 2. The connecting side 2 is slid over the mouthpiece 10, and the rubber ring is pushed from the top of adaptor connecting side 2 over the mouthpiece of the inhaler 10. In addition, the interior of the attachment side, in which the attachment side of an interior cone 9 is located, and the inhaler mouthpiece, link end to end, forming a seal. Thus, there is no ridge on which particles can coalesce, formed between the adaptor and the inhaler mouthpiece 10.

Further, in Figure 3 a conical wall 8 is formed on the inside of the transition section 4. The interior wall forms a smooth transition between the inhaler mouthpiece and the adaptor, thereby allowing the particles to move through the inhaler mouthpiece with minimum turbulence. As in the above embodiments, aerated particles only enter and pass through the adaptor when the inhaler is stimulated to pass medication. Thus, the adaptor does not act to hold-up flow of medication.

The mouthpiece side in the adaptor of Figure 3 could be shaped, e.g., according to the first or second embodiment.

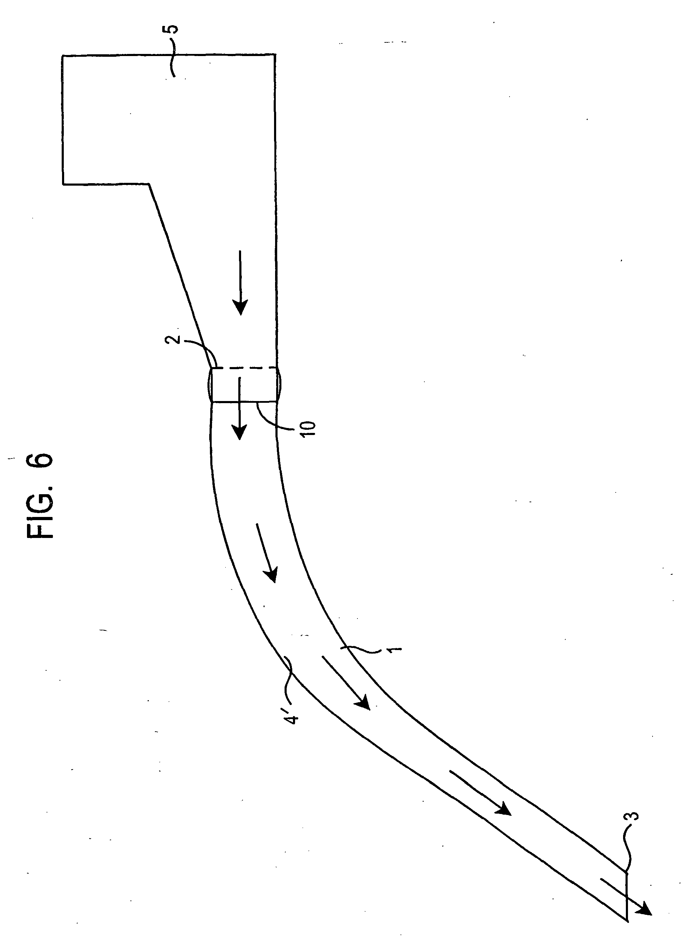

A fourth embodiment is shown in Figure 6. As in the other embodiments, the fourth embodiment consists of a connecting side 2, a transition section 4', and a mouthpiece side 3. However, in the Figure 6 embodiment, the transition section 4' comprises an elongate, curved, generally tubular section of constantly narrowing cross-section. The cross-sectional shape of the transition section 4' preferably is of airfoil shape, so as to minimize turbulence whereby to keep the aerated particles from colliding and coagulating. Further, the extension should be long enough to allow a care-giver to administer medicine from the inhaler to a person lying in a prone or semi-prone position, i.e., without the patient having to sit up or hold the inhaler. Finally, as in the other embodiments, the connecting means facilitates the immediate passage or medication between the inhaler mouthpiece and the oral contact means of the adaptor, without acting as a holding chamber for the medication.

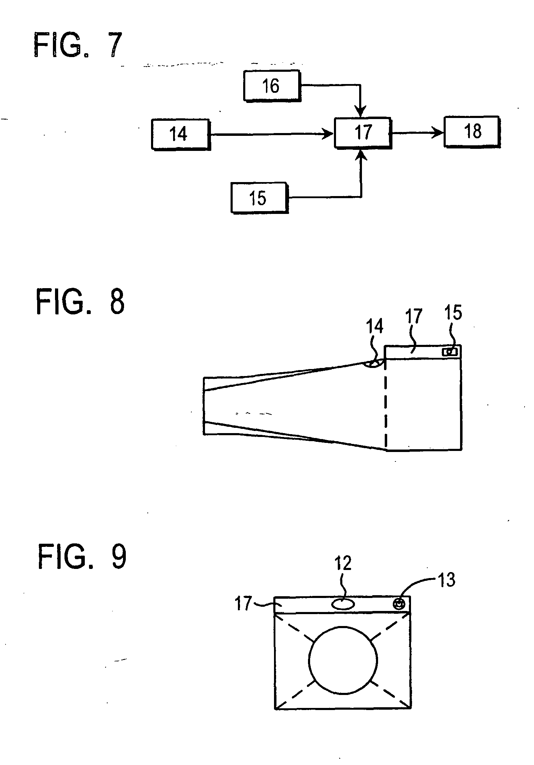

In an embodiment of the present invention, illustrated in Figures 7-9, the mouthpiece adaptor or DPI itself includes electronic means to teach the patient to retrieve a proper breathing pattern. Such electronic means includes a sensor 14, an on/off switch 15, a power source 16, actuation controller 17, noise or light generating means 18, a light 12, and optionally a speaker 13. As shown in Figure 7, the actuation controller 17 supplies power to the noise and light generating means 18 only when the sensor 14 detects a patient is inhaling and the power switch 15 is on. Upon receiving Like the mouthpiece side in the adaptor of Figure 1, the mouthpiece side as illustrated in Figure 4, is child-sized, i.e., having cross-sectional dimensional area from 1mm² to 200mm². This design, like the round or cylindrical mouthpiece side, also has advantages. Because the shape of the mouthpiece side here is the shape of the inhaler mouthpiece, airflow is concentrated with minimum turbulence.

power, the noise or light generating means 18 determines the strength and frequency of the inhalations, and generates light and sound based upon these values. If the patient is not attaining the proper breathing pattern for a particular device, the noise and light generating means 18 causes a light 12 to turn red, which signals to the patient to change his or her breathing pattern to conform to the protocols of the device. Correlating sounds may also be produced by the light/sound generating means 18 and output to the speaker 13. Once the proper breathing pattern is attained, the light 12 on the adaptor 1 changes from red to green, and/or the sound/light generating means 18 informs the patient that they have attained the proper breathing pattern.

In another embodiment of the present invention the electronic means to teach the patient to inhale properly is integral to the inhaler.

## Claims

1. A dry powder inhaler (5) for delivering medication to the respiratory tract of a patient, wherein the inhaler includes:
a sensor (14) detecting that the patient is inhaling, an on/off switch (15), a power source (16), an actuation controller (17) supplying power to a noise or light generating means (18) only when the sensor (14) detects a patient is inhaling and the power switch (15) is on, a light (12) and the noise or light generating means (18) being for determining strength and frequency of inhalations, and for generating light and sound based upon a value of the determined strength and frequency of inhalations, for informing a patient that they have attained a proper breathing pattern.

2. The inhaler according to claim 1, **characterized in that** it comprises a speaker (13).

3. The inhaler according to any of the preceding claims, **characterized in that** the light (12) changes between red and green.

4. The inhaler according to any of claims 1-3, further **characterized by** a mouthpiece adaptor (1) having an attachment side (2) connected to a mouthpiece (10) of the inhaler (5), a mouthpiece side (3) of reduced size relative to the inhaler mouthpiece (10); and a transition section (4) between said attachment side (2) and mouthpiece side (3) and the adapter is tapered from the attachment side (2) to the mouthpiece side (3).

## Patentansprüche

1. Trockenpulverinhalator (5) zur Medikamentenabgabe in die Atemwege eines Patienten, wobei der Inhalator Folgendes aufweist:
einen Sensor (14), um zu erfassen, dass der Patient gerade inhaltiert, einen Ein-/Aus-Schalter (15), eine Stromquelle (16), ein Betätigungssteuerorgan (17), das ein ein Geräusch oder Licht erzeugendes Mittel (18) nur dann mit Strom versorgt, wenn der Sensor (14) erfasst, dass ein Patient gerade inhaliert und der Leistungsschalter (15) eingeschaltet ist, eine Leuchte (12), wobei das ein Geräusch oder Licht erzeugende Mittel (18) dazu vorgesehen ist, die Stärke und Häufigkeit der Inhalationen zu bestimmen und Licht oder ein Geräusch anhand eines Werts der bestimmten Stärke und Häufigkeit der Inhalationen zu erzeugen, um einen Patienten darüber zu informieren, dass er eine korrekte Atmungsweise erreicht hat.

2. Inhalator nach Anspruch 1, **dadurch gekennzeichnet, dass** er einen Lautsprecher (13) aufweist.

3. Inhalator nach einem der vorhergehenden Ansprüche, **dadurch gekennzeichnet, dass** die Leuchte (12) zwischen rot und grün wechselt.

4. Inhalator nach einem der Ansprüche 1 bis 3, ferner **gekennzeichnet durch** einen Mundstückadapter (1) mit einer Befestigungsseite (2), die mit einem Mundstück (10) des Inhalators (5) verbunden ist, einer Mundstückseite (3) mit einer in Bezug auf das Inhalatormundstück (10) verringerten Größe, und einem Übergangsabschnitt (4) zwischen der Befestigungsseite (2) und der Mundstückseite (3), wobei der Adapter von der Befestigungsseite (2) zur Mundstückseite (3) verjüngt ist.

## Revendications

1. Inhalateur (5) de poudre sèche pour administrer un médicament dans les voies respiratoires d'un patient, l'inhalateur comportant:
un capteur (14) pour détecter que le patient fait une inhalation, un interrupteur de marche/arrêt (15), une source de courant (16), un organe de commande d'actionnement (17) alimentant un moyen (18) de génération de bruit ou de lumière en électricité uniquement lorsque le capteur (14) détecte qu'un patient fait une inhalation et que l'interrupteur de puissance (15) est à l'état de marche, et une lampe (12), le moyen (18) de génération de bruit ou de lumière étant prévu pour déterminer l'intensité et la fréquence des inhalations et pour générer de la lumière et du bruit sur la base d'une valeur de l'intensité et de la fréquence déterminées des inhalations, afin d'informer un patient qu'il a atteint un mode de respiration approprié.

2. Inhalateur selon la revendication 1, **caractérisé en ce qu'**il présente un haut-parleur (13).

3. Inhalateur selon l'une des revendications précédentes, **caractérisé en ce que** la lampe (12) change entre rouge et vert.

4. Inhalateur selon l'une des revendications 1 à 3, en outre **caractérisé par** un adaptateur d'embout (1) présentant un côté d'attache (2) relié à un embout (10) de l'inhalateur (5), un côté d'embout (3) de taille réduite par rapport à l'embout (10) de l'inhalateur, et un tronçon de transition (4) entre le côté d'attache (2) et le côté d'embout (3), l'adaptateur étant conique depuis le côté d'attache (2) vers le côté d'embout (3).
